(19) Europäisches Patentamt
European Patent Office
Office européen des brevets

(11) Veröffentlichungsnummer: **0 212 479 A1**

(12) # EUROPÄISCHE PATENTANMELDUNG

(21) Anmeldenummer: **86110950.2**

(22) Anmeldetag: **08.08.86**

(51) Int. Cl.⁴: **A 23 K 1/16**, A 23 K 1/18, C 07 D 211/72, C 07 D 223/12, C 07 D 225/02

(30) Priorität: **20.08.85 DE 3529692**

(43) Veröffentlichungstag der Anmeldung: **04.03.87 Patentblatt 87/10**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Kabbe, Hans-Joachim, Dr., Walter-Flex-Strasse 16, D-5090 Leverkusen 1 (DE)**
Erfinder: **Berschauer, Friedrich, Dr., Claudiusweg 9, D-5600 Wuppertal 1 (DE)**
Erfinder: **Scheer, Martin, Dr., Herberts-Katernberg 7, D-5600 Wuppertal 1 (DE)**
Erfinder: **de Jong, Anno, Dr., Stockmannsmühle 46, D-5600 Wuppertal 1 (DE)**

(54) **Verwendung von Lactamamiden als Leistungsförderer bei Tieren.**

(57) Die vorliegende Erfindung betrifft die Verwendung von Lactamamiden der Formel I

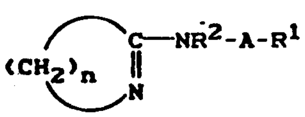

I

in welcher
n für 3 bis 6 steht,
A für eine direkte Bindung oder für eine gegebenenfalls substituierte Alkylenbrücke steht,
$R^1$ für gegebenenfalls substituiertes Aryl steht,
$R^2$ für Wasserstoff oder Alkyl steht
sowie ihre physiologisch verträglichen Salze mit Säuren als Leistungsförderer bei Tieren.

Die Verbindungen der Formel I können auch in ihrer isomeren Form der Formel Ia

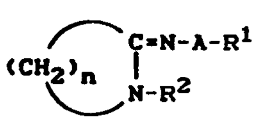

Ia

vorliegen.

Die Erfindung betrifft außerdem neue Lactamamide und Verfahren zu ihrer Herstellung.

EP 0 212 479 A1

0212479

BAYER AKTIENGESELLSCHAFT 5090 Leverkusen, Bayerwerk
Konzernverwaltung RP
Patentabteilung Rt/Ke-c

# Verwendung von Lactamamiden als Leistungsförderer bei Tieren

Die vorliegende Erfindung betrifft die Verwendung von Lactamamiden als Leistungsförderer bei Tieren, neue Lactamamide und Verfahren zu ihrer Herstellung.

Lactamamide sind bereits bekannt geworden. Sie werden zum Teil als Pestizide zur Bekämpfung von Insekten, Akariden aber auch als Pharmazeutica zur Verbindung der Hypertonie, Übersäuerung des Magens oder als Anticoagulantien eingesetzt (DE-P 1 167 816, 2 009 019, US-P 3 959 479).

In keinem Fall ist jedoch bekannt geworden, daß Lactamamide als Leistungsförderer bei Tieren eingesetzt werden können.

1. Es wurde gefunden, daß sich Lactamamide der Formel I

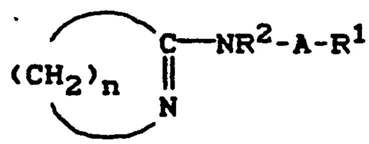

I

in welcher

n für 3 bis 6 steht,

A für eine direkte Bindung oder für eine gegebenenfalls substituierte Alkylenbrücke steht,

$R^1$ für gegebenenfalls substituiertes Aryl steht,

$R^2$ für Wasserstoff oder Alkyl steht

sowie ihre physiologisch verträglichen Salze mit Säuren als Leistungsförderer bei Tieren eignen.

Die Verbindungen der Formel I können auch in ihrer isomeren Form der Formel Ia

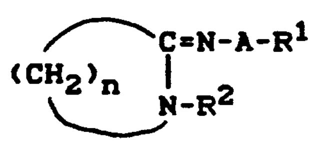

Ia

vorliegen.

2. Es wurden die neuen Lactamamide der Formel II

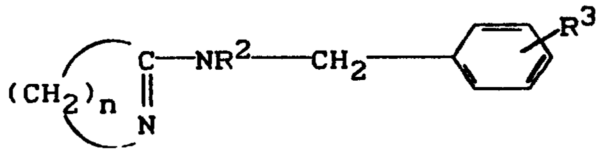

II

in welcher

n für 4 oder 5 steht,

$R^2$ für Wasserstoff oder Alkyl steht,

$R^3$ für einen oder mehrere, gleiche oder verschiedene Reste aus der Gruppe Wasserstoff, Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Chlorfluorethyl, Halogenalkoxy

mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylmercapto; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen, wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbe-

0212479

sondere 1 oder 2 Kohlenstoffatomen je Alkylgruppe, wie Methylamino, Methyl-ethyl-amino, n- und i-Propylamino und Methyl-n-Butylamino; Formyl, Carboxyl; Alkylcarbonyl mit 1 bis 4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; wobei die Alkylgruppe durch 1 bis 5 Fluoratome substituiert sein kann; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio die ihrerseits wieder substituiert sein können, steht,

sowie die Salze der Verbindungen der Formel II mit Säuren gefunden. Die Verbindungen der Formel II können auch in ihrer isomeren Form der Formel IIa

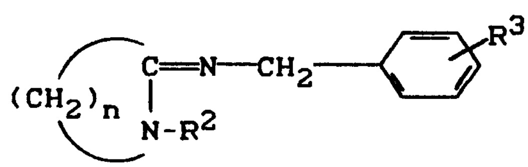

IIa

vorliegen.

3. Es wurde gefunden, daß man die Lactamamide der Formel II

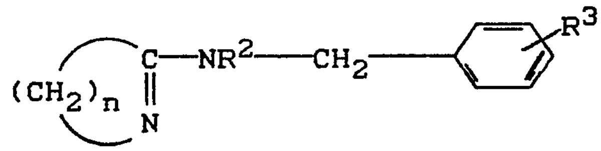

II

in welcher

n für 4 oder 5 steht,

$R^2$ für Wasserstoff oder Alkyl steht,

$R^3$ für einen oder mehrere, gleiche oder verschiedene Reste aus der Gruppe Wasserstoff, Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Chlorfluorethyl, Halogenalkoxy

mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylmercapto; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugweise Fluor oder Chlor, insbesondere Fluor stehen, wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Formyl; Carboxyl; Alkylcarbonyl mit 1 bis 4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl, wobei die Alkylgruppe durch 1 bis 5 Fluoratome substituiert sein kann; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio die ihrerseits wieder substituiert sein können, steht,

erhält, indem man

a) Verbindungen der Formel III

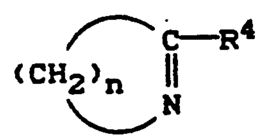

III

in welcher

n für 4 oder 5 steht,

$R^4$ für Halogen, Alkoxy, Alkylthio steht,

mit Aminen der Formel IV

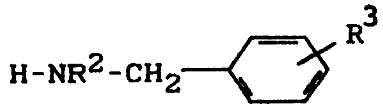 IV

in welcher

$R^2$, $R^3$ die oben angegebene Bedeutung haben,

umsetzt, oder

b) indem man Lactame der Formel V

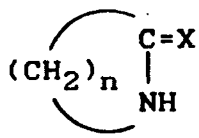 V

in welcher

n für 4 oder 5,

X für O, S oder NH steht,

mit Aminen der Formel IV

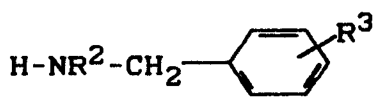 IV

in welcher

$R^2$, $R^3$ die oben angegebene Bedeutung haben,

in Gegenwart eines Kondensationsmittels umsetzt.

Es war überraschend, daß Verbindungen der Formel I hervorragende leistungsfördernde Wirkung bei Tieren besitzen. Die seither bekannten Eigenschaften der bekannten Lactamamide ließen keinen Schluß auf eine solche Verwendung zu.

Lactamamide der Formel I sind teilweise bekannt oder lassen sich analog zu bekannten verfahren herstellen (vgl. DE-OS 2 029 297, 1 167 816, 2 009 019; US-P 3 959 479; BE-P 814 114).

Bevorzugt werden Lactamamide der Formel I verwendet, in welcher

n für 4 oder 5 steht,

$R^2$ für Wasserstoff oder $C_{1-4}$-Alkyl steht,

$R^1$ für Phenyl oder Naphthyl steht, die gegebenenfalls durch einen oder mehrere, gleiche oder verschiedene der folgenden Reste substituiert sind: Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen,

wobei die Halogenatome gleich oder verschieden sind und als Halogenatome, vorzugsweise Fluor, Chlor oder Brom, insbesondere Fluor stehen, wie Trifluormethyl, Chlorfluorethyl, Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylmercapto; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugweise Fluor oder Chlor, insbesondere Fluor stehen, wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Formyl;

Carboxyl; Alkylcarbonyl mit 1 bis 4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl, wobei die Alkylgruppe durch 1 bis 5 Fluoratome substituiert sein kann; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio die ihrerseits wieder substituiert sein können, steht,

A für eine Alkylenbrücke mit 1 bis 3 C-Atomen, die gegebenenfalls ein oder mehrfach durch $C_{1-4}$-Alkyl substituiert ist, steht.

Besonders bevorzugt werden Lactamamide der Formel I verwendet, in welcher

n für 4 oder 5 steht,

$R^2$ für Wasserstoff steht,

$R^1$ für Phenyl steht, das durch einen oder mehrere gleiche oder verschiedene der folgenden Reste substituiert ist:
Alkyl mit 1 bis 4 Kohlenstoffatomen, Alkoxy mit 1 bis 4 Kohlenstoffatomen, Chlor, Fluor und Brom, Halogenalkyl mit 1 oder 2 Kohlenstoffatomen und 1 bis 5 Halogenatomen wie Trifluormethyl, Pentafluorethyl, Halogenalkoxy mit 1 oder 2 C-Atomen wie Trifluormethoxy oder Pentafluorethoxy, Alkylmercapto- und Alkylsulfonyl mit 1 oder 2 C-Atomen, wobei die Alkylgruppe 1 bis 5 Fluoratome enthalten kann, Cyano, Nitro, Methylen- und Ethylendioxy, die gegebenenfalls durch Halogen, insbesondere Fluor substituiert sein können

A für die Brücken $-CH_2-$, $-C_2H_4-$, $-\overset{\displaystyle CH_3}{\overset{|}{C}}H-CH_2-$, $-C(CH_3)_2CH_2-$ steht.

Insbesondere seien genannt Verbindungen der Formel I, in welcher

n für 5 steht,

$R^2$ für Wasserstoff steht,

$R^1$ für Phenyl steht das durch einen oder mehrere, gleiche oder verschiedene der folgenden Reste substituiert ist: Halogen, insbesondere Chlor, $C_{1-2}$-Halogenalkyl, insbesondere Trifluormethyl, $C_{1-4}$-Alkyl, insbesondere Methyl, Ethyl, Propyl, $C_{1-4}$-Alkoxy, insbesondere Methoxy, Ethoxy, Methylendioxy oder Ethylendioxy die gegebenenfalls durch Halogen, insbesondere Fluor substituiert sind.

Im einzelnen seien folgende Verbindungen der Formel I genannt:

| n | $R^2$ | A | $R^1$ |
|---|---|---|---|
| 5 | $CH_3$ | $-(CH_2)_2-$ | m-$CF_3$-Phenyl |
| 4 | H | $-(CH_2)_3-$ | p-Cl-Phenyl |
| 5 | H | $-(CH_2)_1-$ | 3.4-$Cl_2$-Phenyl |
| 5 | H | $-(CH_2)_1-$ | 3-$CF_3$S-Phenyl |
| 4 | H | $-(CH_2)_1-$ | 3-$CF_3$S-Phenyl |
| 3 | H | $-(CH_2)_1-$ | 3-$CF_3$-Phenyl |
| 5 | H | $-(CH_2)_1-$ | Phenyl |

Als Säuren mit denen physiologisch verträgliche Salze der Verbindungn der Formel I gebildet werden können seien bevorzugt genannt: organische Carbonsäuren wie Essigsäure, Milchsäure, Apfelsäure, Ascorbinsäure, Maleinsäure, Citronensäure, Bernsteinsäure, Weinsäure, Glutarsäure, Benzoesäure, 2-Hydroxynaphthalincarbonsäure-(3), Salicylsäure, Embonsäure: Sulfonsäuren wie Methansulfonsäure, Ethansulfonsäure, Trifluormethansulfonsäure, Benzolsulfonsäure, Napthalindisulfonsäure-(1,5); anorganische Säuren wie Salzsäure, Bromwasserstoffsäure, Schwefelsäure, Phosphorsäure, Salpetersäure.

Besonders bevorzugt seien die folgenden Säuren genannt: Essigsäure, Bernsteinsäure, Salicylsäure, Trifluormethansulfonsäure, Benzolsulfonsäure, Schwefelsäure und Salpetersäure.

Insbesondere seien die folgenden Säuren genannt: Milchsäure, Maleinsäure, Citronensäure, Weinsäure, Methansulfonsäure, Naphthalindisulfonsäure, Salzsäure und Phosphorsäure.

Von den neuen Lactamamiden der Formel II sind bevorzugt diejenigen in welcher

n für 5 steht,

$R^2$ für Wasserstoff steht,

$R^3$ für einen oder mehrere, gleiche oder verschiedene der Reste Halogen, insbesondere Chlor, $C_{1-2}$-Halogenalkyl, insbesondere Trifluormethyl, $C_{1-4}$-Alkyl, insbesondere Methyl, Ethyl, Propyl, $C_{1-4}$-Alkoxy, insbesondere Methoxy oder Ethoxy, Methylendioxy oder Ethylendioxy, die gegebenenfalls durch Halogen insbesondere Fluor substituiert sind, steht, wobei besonders die Verbindungen hervorgehoben wird, bei denen mindestens einer der Reste $R^3$ für Trifluormethyl steht.

Die neuen Lactamamide lassen sich durch Umsetzung der Verbindungen der Formel III mit Verbindungen der Formel IV herstellen. Die Reaktion läßt sich z.B. durch folgendes Formelschema wiedergeben:

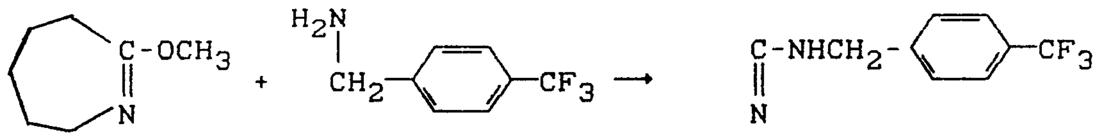

Als Ausgangsverbindungen werden bevorzugt diejenigen eingesetzt in denen die Substituenten die bei den Verbindungen der Formel I und II genannten bevorzugten Bedeutungen haben. Im einzelnen seien folgende Verbindungen der Formel III genannt:

2-Methoxytetrahydroazepin,
2-Ethoxytetrahydroazepin,
2-Isopropoxytetrahydroazepin,
2-Methylmercaptotetrahydroazepin,
2-Chlortetrahydroazepin,
2-Methoxytetrahydropyridin,
2-Butoxytetrahydropyridin,
2-Methylmmrcaptotetrahydropyridin,
2-Bromtetrahydropyridin,
2-Methoxydihydropyrrol,
2-Propylmercaptodihydropyrrol,
2-Chlordihydropyrrol.

Im einzelnen seien folgende Verbindungen der Formel IV genannt:

Anilin, 2-, 3- und 4-Chloranilin, 2-, 3- und 4-Methylanilin, 2-, 3- und 4-Methoxyanilin, 2-, 3- und 4-Butoxyanilin, 2-, 3- und 4-Methylmercaptoanilin, 2-, 3- und 4-Trifluormethylanilin, 3-Chlor-4-trifluormethylanilin, 2-Methoxy-4-chloranilin, 2-Methyl-3-methoxyanilin, sowie die entsprechenden Benzylamine, α-Phenethylamine, β-Phenethylamine und γ-Phenylpropylamine.

Die Verbindungen der Formeln III und IV sind bekannte Verbindungen der organischen Chemie. Sie lassen sich nach an sich bekannten Methoden herstellen.

Die Umsetzung der Verbindungen der Formel III mit den Verbindungen der Formel IV erfolgt bevorzugt in Gegenwart von Verdünnungsmitteln die gegen die Ausgangsstoffe III und IV sowie die Endprodukte I inert sind. Als solche seien genannt: Kohlenwasserstoffe wie Petrolether, Cyclohexan, Methylcyclohexan, Benzol, Toluol, Xylol, Trimethylbenzol; Chlorbenzol; Ether wie Diethylether, Dibutylether, Tetrahydrofuran, Dioxan, Glycoldimethylether; Alkohole wie Methanol, Ethanol, Isopropanol.

Die Umsetzung erfolgt bei 20°C bis 200°C, bevorzugt von 80°C bis 160°C.

Die Umsetzung erfolgt bei Normaldruck.

Die Verbindungen der Formel III und IV werden in äquimolarem Verhältnis zusammengegeben. Ein Überschuß der einen oder anderen Verbindung bringt keine wesentlichen Vorteile. Bevorzugt werden die Verbindungen III und IV im Verhältnis 1:0,5 bis 1:2 eingesetzt.

Die Aufarbeitung erfolgt nach beendeter Reaktion bevorzugt durch destillierte Trennung der Reaktionskomponenten und Lösungsmittel.

Die neuen Lactamamide lassen sich auch durch Umsetzung der Verbindungen der Formel V mit Aminen der Formel IV in Gegenwart vom Kondensationsmitteln herstellen. Die Reaktion läßt sich z.B. durch folgendes Formelschema wiedergeben:

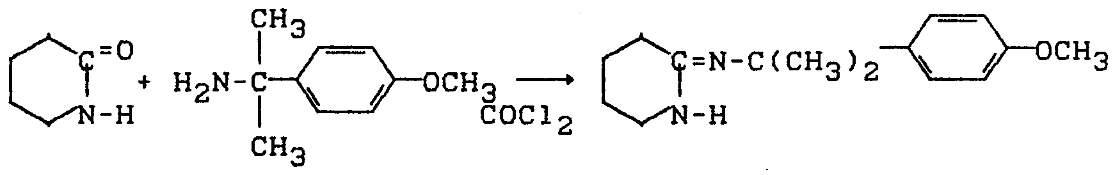


Als Ausgangsverbindungen werden bevorzugt diejenigen eingesetzt, in denen die Substituenten die bei den Verbindungen der Formel I und II genannten bevorzugten Bedeutungen haben. Im einzelnen seien die bei Verfahren a) genannten Amine der Formel IV genannt. Im einzelnen seien die folgenden Verbindungen der Formel V genannt: Butyrolactam, 5-Aminopentansäurelactam, Caprolactam.

Die Verbindungen der Formel V sind bekannte Verbindungen der organischen Chemie. Sie lassen sich nach an sich bekannten Verfahren herstellen.

Die Umsetzung der Verbindungen der Formel V mit den Verbindungen der Formel IV erfolgt bevorzugt in Gegenwart eines Verdünnungsmittels. Als solche seien genannt: Kohlenwasserstoffe wie Petrolether, Cyclohexan, Methylcaclohexan, Benzol, Toluol, Xylol, Trimethylbenzol, Chlorbenzol; Ether wie Diethylether, Dibutylether, Tetrahydrofuran, Dioxan, Glycoldimethylether.

Als Kondensationsmittel seien genannt: Phosgen, Phosphoroxychlorid, Thionylchlorid.

Die Umsetzung erfolgt bevorzugt indem man Verbindungen der Formel V und IV im Verdünnungsmittel vorlegt und das Kondensationsmittel zutropft.

Die Umsetzung erfolgt bei -20°C bis +100°C, bevorzugt bei 0°C bis 50°C.

Die Umsetzung erfolgt bei Normaldruck.

Die Verbindungen V und IV werden im Verhältnis 1:0,5 bis 1:2 eingesetzt. Bevorzugt von 1:0,8 bis 1:1,2. Das Kondensationsmittel wird bezogen auf die Verbindung V im Verhältnis 1:0,5 bis 1:2 eingesetzt, bevorzugt 1:1.

Die Aufarbeitung erfolgt nach beendeter Reaktion indem man das Reaktionsgemisch mit Wasser versetzt, die Lösung durch Zugabe von Natronlauge oder Sodalösung neutralisiert oder schwach basisch macht, das Endprodukt mit einem mit Wasser nicht mischbaren Lösungsmittel ausschüttelt und durch Destillation reinigt.

Die Wirkstoffe werden als Leistungsförderer bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet.

 0212479

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Puten, Gänse, Enten, Tauben, Fische wie z.B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier- und Aquarienfische z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Wachstums- und Leistungsphase, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können eimalig verarbeitet werden. Die Wirkstoffe können aber auch während der ganzen oder während eines Teils der Wachstums- und Leistungsphase temporär oder kontinuierlich verabreicht werden.

Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form.

Die Wirkstoffe können in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind:
z.B. Antibiotika wie Tylosin und Virginiamycin.
Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid.
Vitamine sind z.B. Vitamin A, Vitamin $D_3$, Vitamin E, B-Vitamine, Vitamin C.
Nicht-Protein-Verbindungen sind z.B. Biuret, Harnstoff.
Farbstoffe sind z.B. Carotinoide wie Citranaxanthin, Zeaxanthin, Capsanthin.
Antioxidantien sind z.B. Aethoxyquin, Butylhydroxy-Toluol.
Aromastoffe sind z.B. Vanillin.
Emulgatoren sind z.B. Ester der Milchsäure, Lecithin.
Fließhilfsstoffe sind z.B. Natriumstearat, Calciumstearat.
Konservierungsstoffe sind z.B. Zitronensäure, Propionsäure.
Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäure z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung die eine ausgewogene Ernährung hinsichtlich der Energie- und Proteinversorgung sowie der Versorung mit Vitaminen, Mineralsalzen und Spurenelementen sicherstellen.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:
600 I.E. Vitamin A, 100 I.E. Vitamin, $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $So_2$ x $H_2O$, 140 mg Zn $So_4$ x 7 $H_2O$, 100 mg Fe $SO_4$ x 7 $H_2O$ und 20 mg Cu $So_4$ x 5 $H_2O$.

2,5 g Wirkstoff-Prämix enthalten z.B. 10 mg Wirkstoff, 1 g DL-Methion, Rest Sojabohnenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

0212479

## Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt.

Es werden die aus der Tabelle ersichtlichen Ergebnisse erhalten:

0212479

Tabelle a

Ratten Fütterungsversuch

| Wirkstoff Bsp.Nr. | Dosis ppm | Tierzahl | Zuwachs |
|---|---|---|---|
| Kontrolle | | | 100 |
| 14 (Maleinat) | 25 | 96 | 117 |
| 1 | 25 | 54 | 111 |
| 3 | 25 | 18 | 107 |
| 9 | 25 | 42 | 104 |
| 10 | 25 | 30 | 107 |
| 13 | 25 | 54 | 112 |

Beispiel 1

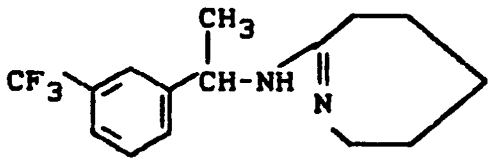

8,5 g 2-Methoxytetrahydroazepin und 9,5 g 2-(3-Trifluormethylphenyl)-ethylamin werden in 70 ml Xylol 10 Stunden auf Rückflußtemperatur (ca. 140°C) erhitzt. Anschließend engt man am Rotationsverdampfer ein und destilliert, wobei 6,1 g eines von 130 bis 135°C bei 20 Pascal siedenden farblosen Öls erhalten werden, das beim Abkühlen erstarrt: Fp. 58 bis 60°C.

Beispiel 2

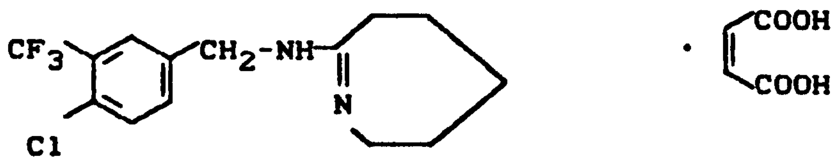

Arbeitet man wie in Beispiel 1, aber mit 3-Trifluormethyl-4-chlorbenzylamin, so erhält man 10 g eines gelben Öls vom Siedepunkt 135 bis 145°C/7 Pascal. Das mit 3,8 g Maleinsäure hergestellte Salz schmilzt bei 110 bis 112°C.

**Beispiel 3**

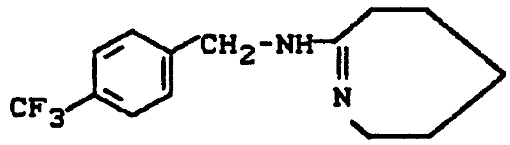

In der gleichen Weise werden mit 8,3 g 4-Trifluormethylbenzylamin 9,3 g eines bei 135 bis 140°C/20 Pascal siedenden Öls erhalten, das bei Raumtemperatur fest wird: Fp. 61 bis 63°C.

**Beispiel 4**

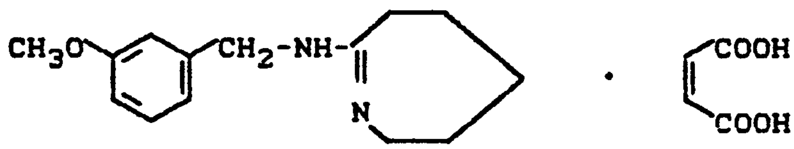

Mit 3-Methoxybenzylamin (6,9 g) erhält man in gleicher Weise wie in Beispiel 1 8,5 g eines Öls, dessen Salz mit Maleinsäure bei 77 bis 79°C schmilzt: Ausbeute: 11,5 g.

**Beispiel 5**

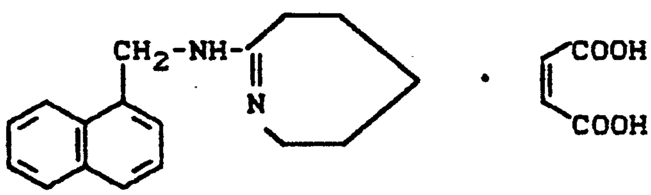

In gleicher weise erhält man aus 7,9 g 2-Aminomethylnaphthalin 12,8 g des Maleinsäure-Salzes: Fp. 133 bis 135°C.

Beispiel 6

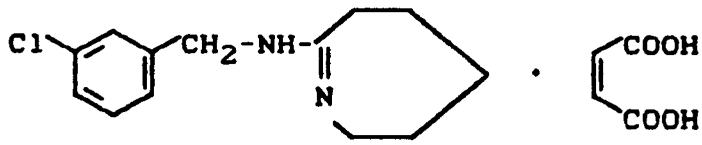

In gleicher Weise erhält man aus 7,1 g 3-Chlorbenzylamin 10,9 g Salz, Fp. 117-9°C.

Beispiel 7

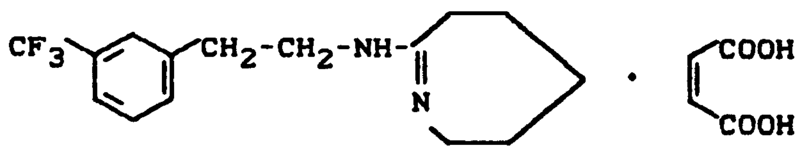

5 g 3-Trifluorphenethylamin und 4,8 g 2-Methoxytetrahydroazepin werden in 70 ml Xylol 10 Stunden auf 140°C erwärmt. Durch Destillation werden 4,8 g 2-(3-Trifluorphenethylamino)-azepin erhalten (Siedepunkt 140 bis 150°C/7 Pascal), die mit 1,96 g Maleinsäure 6,2 g des bei 115 bis 117°C schmelzenden Salzes liefern.

Beispiel 8

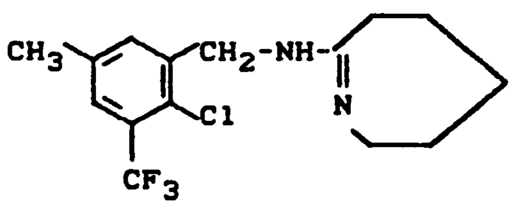

In gleicher Weise wie in Beispiel 1, aber mit 11,2 g 2-Chlor-3-trifluormethyl-5-methylbenzylamin, werden 12,8 g des entsprechenden Lactamamids erhalten; Siedepunkt 150 bis 160°C/27 Pascal, Schmelzpunkt 90 bi 92°C.

Beispiel 9

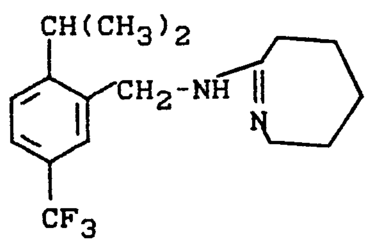

Mit 10 g 2-Isopropyl-5-trifluormethylbenzylamin werden analog Beispiel 1 8,2 g Amidin der obigen Formel erhalten; Siedepunkt: 135bis 145°C/13 Pascal, Schmelzpunkt 84 bis 86°C.

Beispiel 10

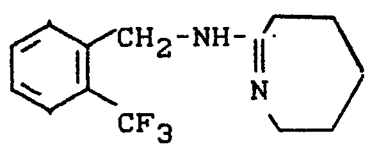

Analog Beispiel 1 werden mit 8,7 g 2-Trifluormethylbenzylamin 7,8 g Lactamamid vom Siedepunkt 143 bis 144°C/ 120 Pascal erhalten; Schmelzpunkt 62 bis 64°C.

Beispiel 11

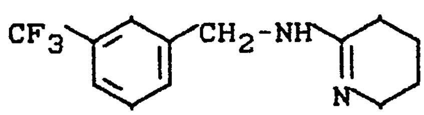

•

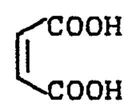

Eine Lösung von 17,5 g 3-Trifluormethylbenzylamin und 14 g 2-Methoxy-3,4,5,6-tetrahydropyridin in 15 ml Toluol wird 8 Stunden auf 110 bis 120°C erhitzt. Durch Destillation

erhält man bei 110 bis 120° C/7 Pascal 18,7 g 2-(3-Trifluormethylbenzylamino)-3,4,5,6-tetrahydropyridin. Das Maleinat schmilzt bei 118 bis 120° C.

Beispiel 12

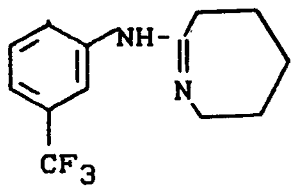

•

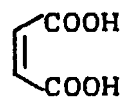

10 g 2-Methoxytetrahydroazepin und 10 g 3-Trifluormethylanilin werden 20 Stunden auf 140 bis 155° C erwärmt. Durch Destillation erhält man 10,3 g eines bei 170 bis 180° C/13 Pascal siedenden Öls, aus dem 12,4 g Maleinsäuresalz der obigen Formel hergestellt werden; Schmelzpunkt 123-5° C.

Beispiel 13

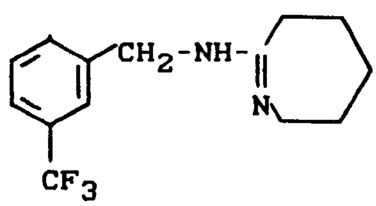

17,5 g 3-Trifluormethylbenzylamin und 14 g 2-Methoxytetrahydroazepin in 80 ml Xylol werden 10 Stunden auf ca. 140° C erwärmt. Destillation liefert 23,2 g 2-(3-Trifluormethylbenzylamino)-tetrahydroazepin; Siedepunkt 150 bis 157° C/53 Pascal.

Beispiel 14

Aus dem Lactamamidin des Beispiel 13 erhält man folgende Salze:

Maleinat: Schmelzpunkt 90 bis 92° C
Suceinat: Schmelzpunkt 80 bis 82° C
Methansulfonat: Schmelzpunkt 85 bis 88° C

Beispiel 15

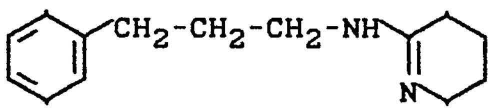

Aus je 10 g 3-Phenylpropylamin und 2-Methylmercaptotetrahydropyridin in 30 ml Chlorbenzol erhält man 11,7 g 2-Phenylpropylaminotetrahydropyridin, das bei 125 bis 130° C/7 Pascal siedet.

Beispiel 16

Eine Lösung von 23 g Caprolactam in 100 ml Toluol wird bei 0° C in 20 Minuten mit 30 g Thionylchlorid versetzt. Man läßt 3 Stunden bei 25° C nachrühren, engt bei unter 30° C am Rotationsverdampfer ein, löst den Rückstand in 100 ml Tetrahydrofuran und gibt langsam 35 g para-Trifluormethylbenzylamin zu. Nach 1 Stunde erwärmt man 2 Stunden auf 60° C, kühlt ab, gießt auf überschüssige Natriumcarbonatlösung und extrahiert mit Toluol. Aus der organischen Phase werden 22 g des gleichen Mediums wie in Beispiel 3 isoliert.

Beispiel 17

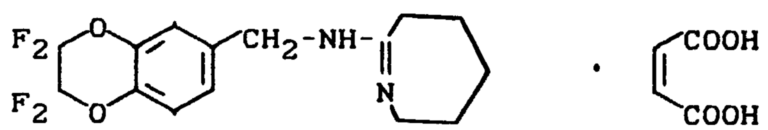

7 g 2-Methoxytetrahydrazepin, 11,9 g 3,4-Tetrafluorethylendioxybenzylamin und 30 ml Xylol werden 10 Stunden auf 140° C erwärmt. Durch Destillation erhält man 9,3 g eines bei 170 bis 180° C/13 Pascal siedenden Öls, das ein bei 126-8° C schmelzendes Maeleinats obiger Formel liefert (Ausbeute: 12,1 g).

Beispiel 18

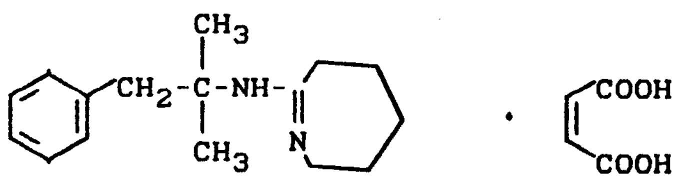

15 g 1,1-Dimethyl-2-phenylethylamin und 18 g 2-Methoxytetrahydroazepin werden 40 Stunden auf 118° C erwärmt. Destillation gibt bei 120 bis 124° C/20 Pascal 4,0 g Lactamamid, aus dem 4,2 g Maleinat der obigen Formel erhalten werden (Schmelzpunkt 116 bis 118° C).

Beispiel 19

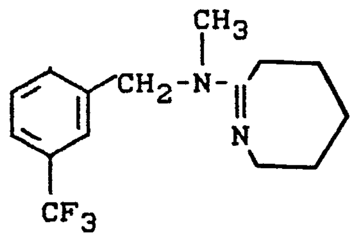

12 g N-Methyl-3-trifluormethylbenzylamin und 12 g 2-Methoxytetrahydroazepin werden 20 Stunden auf 140 bis 150° C erwärmt. Durch Destillation erhält man 9,5 g Lactamamid der obigen Formel als hellgelbes Öl; Siedepunkt 120 bis 125° C/13 Pascal.

## Patentansprüche

1. Verwendung von Lactamamiden der Formel I

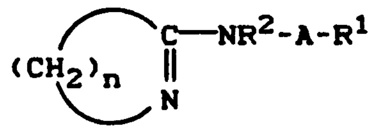

I

in welcher

n für 3 bis 6 steht,

A für eine direkte Bindung oder für eine gegebenenfalls substituierte Alkylenbrücke steht,

$R^1$ für gegebenenfalls substituiertes Aryl steht,

$R^2$ für Wasserstoff oder Alkyl steht

sowie ihre physiologisch verträglichen Salze mit Säuren als Leistungsförderer bei Tieren.

Die Verbindungen der Formel I können auch in ihrer isomeren Form der Formel Ia

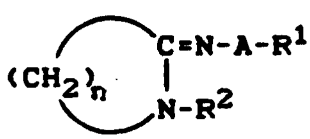

Ia

vorliegen.

2. Mittel zur Leistungsförderung von Tieren, gekennzeichnet durch einen Gehalt an Lactamamiden der Formel I gemäß Anspruch 1.

3. Tierfutter, Trinkwasser für Tiere, Zusätze für Tierfutter und Trinkwasser für Tiere, gekennzeichnet durch einen Gehalt an Lactamamiden der Formel I gemäß Anspruch 1.

4. Verfahren zur Herstellung von Mitteln zur Leistungsförderung von Tieren, dadurch gekennzeichnet, daß man Lactamamide der Formel I gemäß Anspruch 1 mit Streck- und/oder Verdünnungsmitteln versetzt.

5. Verfahren zur Herstellung von Tierfutter, Trinkwasser für Tiere oder Zusätze für Tierfutter und Trinkwasser für Tiere, dadurch gekennzeichnet, daß man Lactamamide der Formel I gemäß Anspruch 1 mit Futtermitteln oder Trinkwasser und gegebenenfalls weiteren Hilfsstoffen vermischt.

6. Lactamamide der Formel II

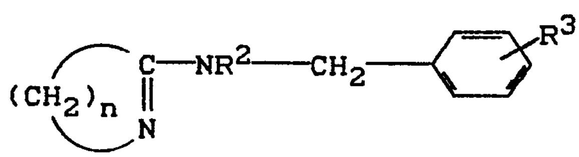

II

in welcher

n für 4 oder 5 steht,

$R^2$ für Wasserstoff oder Alkyl steht,

0212479

$R^3$ für einen oder mehrere, gleiche oder verschiedene Reste aus der Gruppe Wasserstoff, Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Chlorfluorethyl, Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor

stehen, wie Trifluormethylmercapto; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugweise Fluor oder Chlor, insbesondere Fluor stehen, wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Formyl; Carboxyl; Alkylcarbonyl mit 1 bis 4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl, wobei die Alkylgruppe durch 1 bis 5 Fluoratome substituiert sein kann; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio die ihrerseits wieder substituiert sein können, steht,

sowie die Salze der Verbindungen der Formel II mit Säuren. Die Verbindungen der Formel II können auch in ihrer isomeren Form der Formel IIa

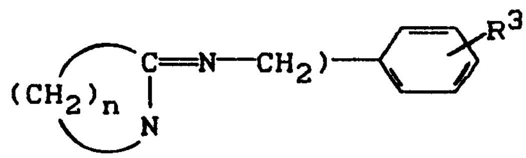

IIa

vorliegen.

7. Verfahren zur Herstellung der Lactamamide der Formel II

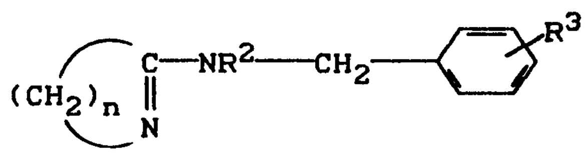

II

in welcher

n für 4 oder 5 steht,

$R^2$ für Wasserstoff oder Alkyl steht,

$R^3$ für einen oder mehrere, gleiche oder verschiedene Reste aus der Gruppe Wasserstoff, Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder

2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Chlorfluorethyl, Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylmercapto; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugweise Fluor oder Chlor, insbesondere Fluor stehen, wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-Butylamino; Formyl, Carboxyl; Alkylcarbonyl mit 1 bis 4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; wobei die Alkylgruppe durch 1 bis 5 Fluoratome substituiert sein kann; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio die ihrerseits wieder substituiert sein können, steht,

dadurch gekennzeichnet, daß man

a) Verbindungen der Formel III

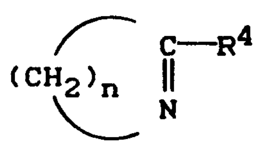

III

in welcher

n für 4 oder 5 steht,

$R^4$ für Halogen, Alkoxy, Alkylthio steht,

mit Aminen der Formel IV

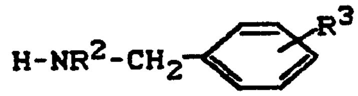

IV

in welcher

$R^2$, $R^3$ die oben angegebene Bedeutung haben,

umsetzt, oder

b) Lactame der Formel V

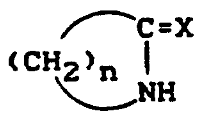

V

in welcher

n für 4 oder 5,

X für O, S oder NH steht,

mit Aminen der Formel IV

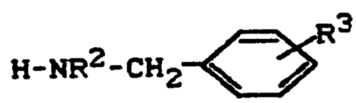

IV

in welcher

$R^2$, $R^3$ die oben angegebene Bedeutung haben,

in Gegenwart eines Kondensationsmittels umsetzt.

8. 2-(3-Trifluormethylbenzyl)-tetrahydroazepin der Formel

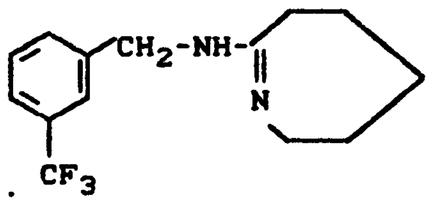

Europäisches Patentamt

# EUROPÄISCHER RECHERCHENBERICHT

0212479
Nummer der Anmeldung

EP 86110950.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CH - A5 - 577 483 (RICHARDSON-MERRELL INC.)<br>* Ansprüche *<br>-- | 1,6,7 | A 23 K 1/16<br>A 23 K 1/18<br>C 07 D 211/72<br>C 07 D 223/12<br>C 07 D 225/02 |
| A | DE - A - 1 670 859 (FARBENFABRIKEN BAYER AG)<br>* Ansprüche 1,2 *<br>-- | 1,6,7 | |
| A | GB - A - 1 413 455 (RICHARDSON-MERRELL INC.)<br>* Ansprüche 1,10 *<br>-- | 1,6,7 | |
| A | GB - A - 579 303 (BOOTS PURE DRUG COMPANY LIMITED)<br>* Ansprüche 1,7 *<br>-- | 1,6 | RECHERCHIERTE SACHGEBIETE (Int. Cl.4) |
| A | US - A - 4 055 561 (J.M. GRISAR et al.)<br>* Spalte 3, Zeile 27 - Spalte 6, Zeile 40 *<br>---- | 1,6,7 | A 23 K<br>C 07 D 211/00<br>C 07 D 223/00<br>C 07 D 225/00 |

Der vorliegende Recherchenbericht wurde für alle Patentansprüche erstellt.

| Recherchenort | Abschlußdatum der Recherche | Prüfer |
|---|---|---|
| WIEN | 12-11-1986 | SLAMA |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument